# EUROPEAN PATENT APPLICATION

(11) **EP 4 674 974 A1**
(43) Date of publication of application: **07.01.2026**
(21) Application number: 24186053.5
(22) Date of filing: 02.07.2024
(51) Int. Cl.: C12Q 1/04, C12Q 1/10, G01N 33/68

(54) **DETECTION OF ENTEROHEMORRHAGIC ESCHERICHIA COLI**

(71) Applicant: Nederlandse Organisatie voor toegepast-natuurwetenschappelijk Onderzoek TNO, 2595 DA 's-Gravenhage (NL)
(72) Inventor: Paauw, Armand, 2595 DA 's-Gravenhage (NL)
(74) Representative: V.O.

(57) **Abstract**

The invention relates to methods for detecting the presence or absence of an enterohemorrhagic *Escherichia coli* (EHEC) in a sample and/or identifying said EHEC. The invention further relates to such methods comprising subjecting the sample or protein therein to mass spectrometry to obtain mass spectrometry data, comparing the obtained mass spectrometry data with reference data for at least one EHEC strain with a specific serotype and optionally one or more non-EHEC strains and detecting the presence or absence of the EHEC.

## Description

### Field of the invention

The invention relates to the field of enterohemorrhagic *Escherichia coli* (EHEC) detection and identification. In particular, the invention relates to such detection and identification using mass spectrometry.

### Background of the invention

*Escherichia coli* (*E. coli*) are a diverse group of bacteria that normally live in the intestines of humans and animals. Shiga toxin-producing *E*. *coli* (STEC) are strains of *E*. *coli* that produce Shiga toxin, a family of related toxins with two major groups, Stx1 and Stx2. Most *E*. *coli* strains are harmless, STEC strains can cause disease in humans but some of these STEC can cause serious illness, which are the enterohemorrhagic *E*. *coli* (EHEC). The Shiga toxin causes damage to the cells in the intestinal wall, called verocytes. The damage can vary in severity and in a small number of cases of infection with this type of STEC hemorrhagic colitis can develop. A small proportion of these cases can progress to hemolytic uremic syndrome (HUS), in which kidney damage occurs.

Traditionally, STEC are distinguished by serotyping of O-polysaccharide antigens and flagellar H-antigens and by the specific toxins. There are more than 200 STEC serotypes, but only a small proportion thereof has been identified by the U.S. Center of Disease Control (CDC) to be the cause of the majority of HUS. These are strain that have the O-types 0157, O26, O45, 0103, O111, O121, and 0145. Since only a small subgroup of STEC forms a health risk, a rapid and robust detection and identification method is needed to specifically and selectively identify these EHEC strains in a sample. Identification of the EHEC in a sample is for instance important to enable e.g. national surveillance and monitor an outbreak or spreading of EHEC infection.

Current methods to identify STEC include O- and H- serotyping and genotyping of genes encoding a Shiga toxin, such as e.g. stx1, stx2, eae and escV genes, and antigen testing for Shiga toxin. For example WO 2015/003171 describes STEC for testing a sample for the present of pathogenic *E*. *coli* which uses the detection of (nucleic acid encoding) (i) ecf and (ii) wzx and/or stx.

No techniques are currently available which allow the rapid and accurate distinction between non-EHEC bacteria and the different EHEC strains of interest (EHEC strains with O-types O157, O26, O45, 0103, 0104, O111, 0121, and/or 0145). Hence, there remains a need in the art for such techniques.

### Summary of the invention

It is an object of the present invention to provide methods for the rapid and accurate detection of enterohemorrhagic *E*. *coli* (EHEC).

The invention therefore provides a method for detecting the presence or absence of an EHEC in a sample, comprising:
- subjecting the sample or protein present therein to a cleavage step to obtain a plurality of peptides,
- comparing the sequences of the obtained peptides with reference data for at least one EHEC strain and optionally one or more non-EHEC strains,
- detecting the presence or absence of the EHEC based on the comparison.

In a further aspect the invention provides a method for the identification of an EHEC in a sample, comprising:
- subjecting the sample or protein present therein to a cleavage step to obtain a plurality of peptides,
- comparing the sequences of the obtained peptides with reference data for at least one EHEC strain and optionally one or more non-EHEC strains,
- identifying the EHEC based on the comparison.

In preferred embodiments, the sample or protein therein or the plurality of peptides is subjected to mass spectrometry to to obtain mass spectrometry data and the obtained mass spectrometry is compared with the reference data. In particular, the obtained mass spectrometry data is used to determine the sequences of the obtained peptides, which sequences are compared with the reference data for at least one EHEC strain and optionally one or more non-EHEC strains.

In a further aspect the invention provides a method for detecting the presence or absence of an EHEC in a sample, comprising:
- subjecting the sample or protein present therein to mass spectrometry to obtain mass spectrometry data,
- comparing the obtained mass spectrometry data with reference data for at least one EHEC strain with a specific serotype and optionally one or more non-EHEC strains,
- detecting the presence or absence of the EHEC based on the comparison.

In a further aspect the invention provides a method for the identification of an enterohemorrhagic EHEC in a sample, comprising:
- subjecting the sample or protein present therein to mass spectrometry to obtain mass spectrometry data,
- comparing the obtained mass spectrometry data with reference data for at least one EHEC strain with specific serotype and optionally one or more non-EHEC strains,
identifying the EHEC based on the comparison.

In a further aspect the invention provides a use of mass spectrometry, preferably liquid chromatography tandem mass spectrometry (LC-MS/MS), to detect the presence or absence of and/or to identify an EHEC in a sample, preferably wherein the sample is subjected to mass spectrometry to obtain mass spectrometry data and the obtained mass spectrometry data is compared with reference data for at least one EHEC strain with a specific serotype and optionally one or more non-EHEC strains.

### Detailed description

As used herein, "to comprise" and its conjugations is used in its non-limiting sense to mean that items following the word are included, but items not specifically mentioned are not excluded. In addition the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.

The word "approximately" or "about" when used in association with a numerical value (e.g. approximately 10, about 10) preferably means that the value may be the given value (e.g. 10), plus or minus 5% of the value (e.g. 10, plus or minus 5%), preferably plus or minus 1% of the value.

The use of the alternative (e.g., "or") should be understood to mean either one, both, or any combination thereof of the alternatives.

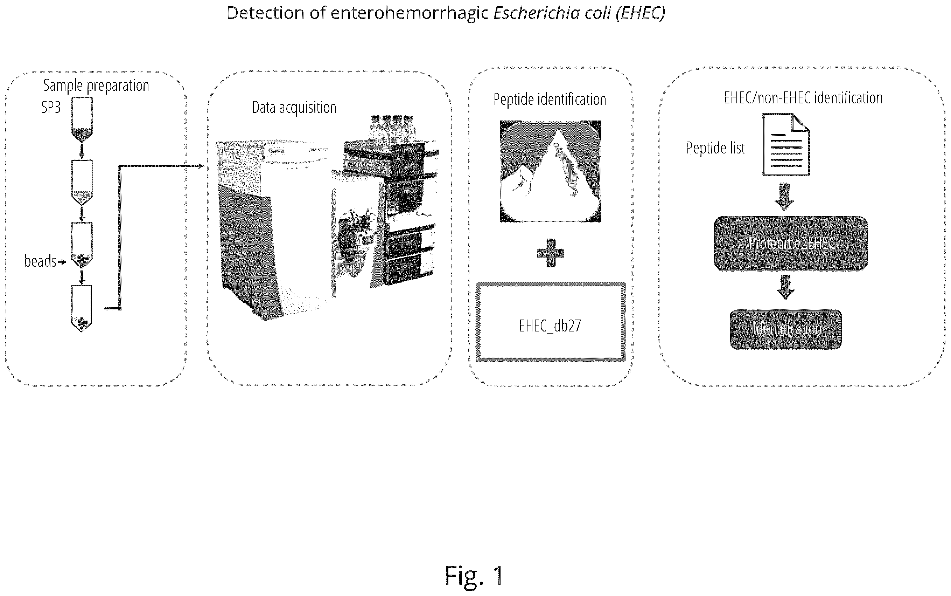

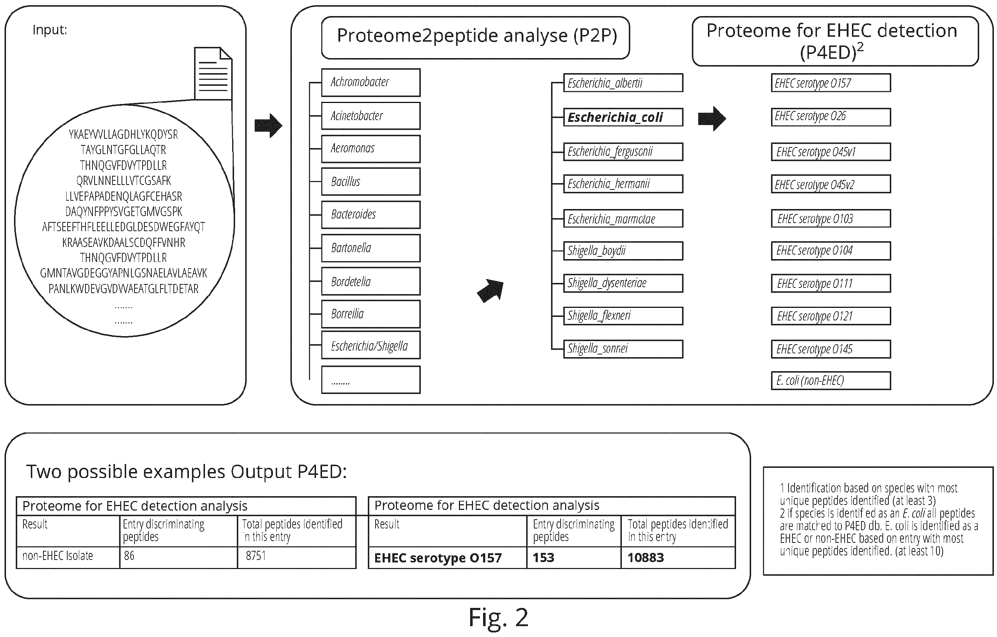

The present inventors have found that it is possible to distinguish specific EHEC strains with specific serotypes from other bacterial species, and thus identify an EHEC strain with a specific serotype based on the proteome of the EHEC strain. It was further found that such distinction can be rapidly made using mass spectrometry. Proteome profiles of each of the EHECs of interest can be made based on the total protein and it was found that these profiles are sufficiently unique to distinguish EHECs with mass spectrometry. It has now also become possible to identify a colony directly as an EHEC strain or non-EHEC strain without to first determine whether or not the colony is an *E*. *coli.* EHEC diagnostics based on proteomics data has not been described before. Moreover, mass spectrometry has mainly been used to distinguish bacteria on the genus-level, followed by a species-level identification step. For instance, Berendsen et al (2017) describes the identification of pathogenic bacteria, e.g. to distinguish *Bacillus anthracis, Brucella abortus, Brucella melitensis, Brucella suis, Burkholderia pseudomallei, Burkholderia mallei, Francisella tularensis* and *Yersinia pestis,* using a genus-level search and a species-level search. The distinction of strains with specific serotypes of a particular bacterial species, such as different EHEC serotypes, differs to a large extent from distinguishing at the genus or species level. The distinction of the proteome of different bacterial genera and species can be represented by a phylogenetic tree-like representation. Contrary, different EHECs are different strains with different serotypes of *E*. *coli* and the proteomes of different *E*. *coli* isolates can be visualized as a cloud, in which the EHEC proteomes are random dots in the cloud. It was therefore surprising that proteomics analysis, such as by mass spectrometry, can nevertheless be used to distinguish the proteome of one specific EHEC from other *E*. *coli* serotypes and from other EHECs. Liquid chromatography tandem mass spectrometry (LC-MS/MS) allows to analyse a sample and identify the bacterial serotype within e.g. 6 hours, which is much faster than currently used methods to identify EHECs. Figures 1 and 2 show schematic representations of an embodiment of the invention.

The invention in one aspects provides a method for detecting the presence or absence of an EHEC in a sample, comprising:
- subjecting the sample or protein present therein to a cleavage step to obtain a plurality of peptides,
- comparing the sequences of the obtained peptides with reference data for at least one EHEC strain with a specific serotype, and optionally one or more non-EHEC strains, and
- detecting the presence or absence of an EHEC based on the comparison.

Also provided is a method for detecting the presence or absence of an EHEC in a sample, comprising:
- subjecting the sample or protein present therein to mass spectrometry to obtain mass spectrometry data,
- comparing the obtained mass spectrometry data with reference data for at least one EHEC strain with a specific serotype, and optionally one or more non-EHEC strain, and
- detecting the presence or absence of an EHEC based on the comparison.

With a method of the invention it is possible to detect the presence or absence of an EHEC in a sample. Preferably the EHEC is detected and identified. The EHEC that is detected and/or identified can be any EHEC strain and serotype. As used herein, "serotype" refers to isolates of *E*. *coli* that are distinguishable in serotype, in particular distinguishable in the O antigen and H antigen. In some preferred embodiments, the EHEC is selected from the serogroup consisting of *E.coli* O157H7, *E.coli* O26H11, *E.colε* O45H2, *E.coli* O103H2, *E*. *coli* O104H4, *E.colε* O111H8, *E.coli* O121H19 and *E.colε* O145H28 and combinations thereof.

In a method of the invention, reference data is used for at least one EHEC strain with a specific serotype. An EHEC strain with a specific serotype thus preferably refers to a strain having a specific O antigen and H antigen, i.e. an EHEC with a specific O antigen en H antigen serotype, for example an *E.colε* O157H7 strain or an *E.colε* O26H11 strain. In some preferred embodiments, the EHEC strain with a specific serotype is selected from the serogroup consisting of *E.coli* O157H7, *E.coli* O26H11, *E.coli* O45H2, *E.coli* O103H2, *E*. *coli* O104H4, *E.colε* O111H8, *E.coli* O121H19 and *E.coli* O145H28 and combinations thereof. Hence, in preferred embodiments, an EHEC strain with a specific serotype is an EHEC O157H7 strain, EHEC O26H11 strain, EHEC O45H2 strain, EHEC O103H2 strain, EHEC O104H4 strain, EHEC O111H8 strain, EHEC O121H19 strain or EHEC O145H28 strain.

In preferred embodiments a method of the invention comprises identifying the EHEC present in the sample. As used herein "identifying the EHEC" means determining the serotype, in particular the O antigen and H antigen serotype, of the EHEC. In particular, the EHEC serotype is selected from *E.coli* O157H7, *E.coli* O26H11, *E.colε* O45H2, *E.colε* O103H2, *E.coli* O104H4, *E.colε* O111H8, *E.coli* O121H19 and *E.colε* O145H28. In some preferred embodiments, detecting the presence of an EHEC comprises identifying the EHEC. Also provided is therefore a method for the identification of an EHEC in a sample, comprising:
- subjecting the sample or protein present therein to a cleavage step to obtain a plurality of peptides,
- comparing the sequences of the obtained peptides with reference data for at least one EHEC strain and optionally one or more non-EHEC strains,
- identifying the EHEC based on the comparison, in particular identifying the EHEC if an EHEC is present or detected to be present.

Also provided is a method for identifying EHEC in a sample, comprising:
- optionally processing the sample to allow it to be subjected to mass spectrometry,
- subjecting the sample or protein present therein to mass spectrometry to obtain mass spectrometry data,
- comparing the obtained mass spectrometry data with reference data for at least one EHEC strain, and optionally one or more non-EHEC strains.
- identifying the EHEC based on the comparison, in particular identifying the EHEC if an EHEC is present or detected to be present.

In some embodiments, the step of identifying an EHEC based on the comparison comprises detecting the presence or absence of an EHEC based on the comparison and, if the presence of an EHEC is detected, identifying the EHEC. Detecting the presence of an EHEC and identifying the EHEC can be performed in a separate steps or in a single step. That is, if an EHEC is identified the presence thereof is also detected in the same step.

In some embodiments, the identification is solely on the basis of the comparison as defined herein. In particular, identification does not comprises genotyping, serotyping and/or detection of biomarkers.

Any method that allows to identify the sequences of a plurality of peptides and compare these sequence with reference data, in particular reference data comprising the sequence of protein and/or peptides of at least one EHEC strain with a specific serotype, and optionally one or more non-EHEC strains, can be used in a method of the invention. Such methods include mass spectrometry, pore peptide sequencing and Next-Generation Protein Sequencing (e.g. Quantum-Si^{™}).

In some preferred embodiments, the sample or protein therein or the plurality of peptides is subjected to mass spectrometry to obtain mass spectrometry data. In further preferred embodiments, the obtained mass spectrometry data is used to determine the sequences of the obtained peptides, i.e. the peptides that are compared with the reference data for at least one EHEC strain and optionally one or more non-EHEC strains. Mass spectrometry is a well-known technique. It is an analytical tool used for measuring the mass-to-charge ratio (m/z) of one or more ionized particles present in a sample. In particular, ionized particles such as atoms, molecules, and clusters are separated by using differences in the ratios of their charges to their respective masses (m/z), which can be used to determine the molecular weight of the particles. Results are typically presented as a mass spectrum, a plot of intensity as a function of the m/z ratio.

In preferred embodiment of a method of the invention, the mass spectrometry is liquid chromatography tandem mass spectrometry (LC-MS/MS). LC-MS/MS combines the separation by liquid chromatography (LC) with mass analysis by mass spectrometry (MS). LC/-MS/MS is particularly suitable for identification and quantification of protein fragments (peptides) in biological samples due to its sensitivity and selectivity.

An LC-MS/MS proteomics based approach has been described for the identification or microorganisms at the genus and species level. Such methods use for the identification of bloodstream infection are for instance described in Berendsen et al. 2017 and Berendsen et al. 2020, which are incorporated herein by reference. However, the present invention for the first time show that LC-MS/MS can be used to distinguish bacterial strains with specific serotypes, in particular *E*. *coli* serotypes.

Typically, LC-MS/MS protein identification and/or quantification involves the extraction of the protein from the sample, enzymatic digestion of the protein, and separation and quantification with liquid chromatography (LC) and tandem mass spectrometry (MS/MS). The fractions that are separated by LC are ionized with electrospray ionization (ESI). The ionized peptides subsequently enter the first quadruple of the mass spectrometer, where a peptide with a specific m/z, the precursor ion, can move to the second quadrupole. Here, the precursor ion is fragmented by using a gas, such as N2, into product ions. The product ions enter the third quadrupole, which selects ions with specific m/z ratios to move to the mass detector. The amino acid sequence of the peptides is determined based on their measured masses, the masses of fragmentation product and comparison with *in silico* enzymatic digests and corresponding predicted fragments of a protein database.

A skilled person is able to selected suitable LC-MS/MS equipment. For instance, the systems used in the Examples herein can be used, i.e. a UHPLC system (UltiMate 3000 Rapid Separation LCnano System, Thermo Fisher connected to a Q Exactive Plus (Q Exactive Plus Orbitrap^{™} Mass Spectrometer, Thermo Scientific) with an EASY-Spray Ion Source (Thermo Fisher)..

Also provided is a use of mass spectrometry, preferably liquid chromatography tandem mass spectrometry (LC-MS/MS), to detect the presence or absence of an EHEC in a sample. Also provided is a use of mass spectrometry, preferably liquid chromatography tandem mass spectrometry (LC-MS/MS), to identity an EHEC in a sample. In preferred embodiments, the sample is subjected to mass spectrometry to obtain mass spectrometry data and the obtained mass spectrometry data is compared with reference data for at least one EHEC strain with a specific serotype and optionally one or more non-EHEC strains.

The sample can be any sample in which *E*. *coli* bacteria can be present. In preferred embodiments, the sample is suspected of comprising *E*. *coli,* in particular STEC and/or EHEC.

In preferred embodiments, the sample is or is obtained from a clinical sample, a food sample or an environmental sample. Preferred examples of clinical samples include a blood or plasma sample, stool sample or urine sample. Preferred examples of environmental samples are a water, soil or sediment sample, e.g. from lakes, rivers, and sewage, manure. Examples of food samples include food ingredients, drinks and beverages, such as (unpasteurized) dairy products, such as milk, yogurt, and cheese, meat samples, fruit samples and vegetable samples, as well as samples, e.g. smears, taken from equipment used in food processing. In some preferred embodiments, the sample is or is obtained from a blood or stool sample.

In some preferred embodiments, the sample is a processed sample, to allow it to determine the sequence of the plurality of peptide and/or to be subjected to mass spectrometry in accordance with the invention. In some embodiments, a method of the invention comprises processing the sample, in particular to allow it to be subjected to mass spectrometry in accordance with the invention. Hence, in some embodiment, the invention provides a method for detecting the presence or absence of an EHEC in a sample, comprising:
- processing the sample to allow it to be subjected to mass spectrometry,
- subjecting the sample or protein present therein to mass spectrometry to obtain mass spectrometry data,
- comparing the mass spectrometry data with reference data for a plurality of EHEC strains with specific serotypes and one or more non-EHEC strains,
- detecting the presence or absence of an EHEC strains based on the comparison.

Processing may include, but is not limited to, isolation of bacteria present in the sample and/or culturing the bacteria. Detection in accordance with the invention is a proteomics-based detection. Hence, in preferred embodiments, the sample comprises bacterial protein, in particular *E*. *coli* protein. In further preferred embodiments, the sample comprises protein from cultured, isolated bacteria, preferably total protein from cultured, isolated bacteria. In some preferred embodiments, the sample comprises bacterial protein, in particular *E*. *coli* protein. The invention entails a proteomics based detection and identification strategy. Hence, the sample preferably bacterial total protein, more preferably *E*. *coli* total protein. "Total protein" as used herein refers to the entire protein component of a sample, in particular of a specific *E. coli* strain with a specific serotype in the context of the present invention.

Methods for the isolation and culturing of bacteria are well known in the art. For instance, the sample can be inoculated and bacteria can be cultured on agar plates or blood agar plates (e.g. comprising culture medium and 5-10% sheep red blood cells), e.g. for at least 16 or 24h at elevated temperature, e.g. at 35°C, or inoculation of culture flasks or blood culture flasks, e.g. for at least 16 or 24h at elevated temperature, e.g. at 35°C.

Prior to subjecting the sample to mass spectrometry, preferably LC-MS/MS, the sample is subjected to a protein digestion or cleavage step. This step results in the generation of a plurality of peptides from the bacterial protein. Techniques for protein digestion are well-known in the art and include enzymatic digestion (e.g. using trypsin) and chemical digestion (e.g. using hydrochloric acid). In preferred embodiments, the sample or protein therein is subjected to enzymatic digestion. Hence, in preferred embodiments, the cleavage step comprises enzymatic digestion. The enzymes used for protein digestions are proteases, in particular endoproteases. Endoproteases hydrolyze peptide bonds between amino acids at specific locations, thereby breaking down the protein into fragments. Preferred examples of enzymes used in enzymatic digestion include, but are not limited to, trypsin, chymotrypsin, Lys-C, GluC, Arg-C, Asp-N and pepsin. Trypsin cleaves after lysine (K) and arginine (R); Chymotrypsin cleaves after aromatic and non-polar amino acids, except leucine; Lys-C cleaves after lysine; Glu-C cleaves after glutamic acid in ammonium bicarbonate buffers and after both glutamic acid and aspartic acid in phosphate buffers; Arg-C cleaves after the positively charged amino acid arginine; Asp-N cleaves at the N-terminal of aspartic acid; pepsin cleaves after the nonpolar amino acids phenylalanine, leucine and tryptophan, and the uncharged polar amino acid tyrosine. In preferred embodiments trypsin is used for enzymatic digestion of protein in the sample.

One or more of the following additional steps can be performed prior to subjecting the sample to mass spectrometry. Non *E*. *coli* proteins, such as proteins from erythrocytes and other host proteins or proteins from culture medium, may be removed from the sample, for instance using saponins and washing steps. In addition, bacteria present in the sample may be ruptured. These processing steps are preformed after isolation and/or culturing of the bacteria and prior to protein isolation and protein digestion.

Whole bacterial cell protein digests can be subjected to mass spectrometry, preferably LC-MS/MS, in accordance with the invention. Alternatively, protein may be isolated and/or purified from the whole bacterial cell digests before subjecting it to mass spectrometry.

In some preferred embodiments, processing of the sample comprises:
- isolating bacteria from the sample and culturing the bacteria, e.g. on an agar plates and/or in a culture flask,
- removal of non-bacterial protein, e.g. washing,
- rupture of bacteria,
- enzymatic digestion of protein, preferably trypsin digestion of protein.

The resulting whole bacterial cell digest, or isolated protein, can subsequently be subjected to methods to determine the sequence of the plurality of peptides. For instance, it can be subjected to mass spectrometry, preferably LC-MS/MS, in accordance with the invention to obtained mass spectrometry data.

A detailed protocol for LC-MS/MS is provided in the Examples herein. In brief, the protein digest is separated using UHPLC coupled to a mass spectrometer using an electrospray ionization source (ESI-source). The MS-spectra can be analysed to identify peptide amino acid sequences and compared to a database with references sequences, e.g. annotated protein sequences in FASTA format that are derived from sequenced genomes on the NCBI FTP server.

A method of the invention comprises comparing the sequences of the obtained peptides with reference data for at least one EHEC strain with a specific serotype, and optionally one or more non-EHEC strains. In some embodiments a method of the invention comprises comparing obtained mass spectrometry data with reference data for at least one EHEC strain with a specific serotype and optionally non-EHEC strains. Hence, the reference data comprises information about the amino acid sequence of protein and/or peptides from at least one EHEC strain with a specific serotype and optionally of one or more non-EHEC strains. The identity of this at least one EHEC strain with a specific serotype is known. Preferably the sequence of the obtained peptides, such as the obtained mass spectrometry data, is compared with reference data for a plurality of EHEC strains with a specific serotype and optionally non-EHEC strains. The identity of each EHEC strain of this plurality of EHEC strains is known. A "plurality of EHEC strains" as used herein refers to at least two EHEC strains with distinct serotypes, in particular distinct O antigen and H antigen serotypes. Preferably the plurality of EHEC strains refers to at least three EHEC strains with distinct serotypes, more preferably at least four, more preferably at least five, more preferably at least six, more preferably at least seven EHEC strains with distinct serotypes, in particular distinct O antigen and H antigen serotypes. The exact number of EHEC strains with distinct serotypes that form the reference data may depend on the situation. E.g. is a sample is suspected to contain one of two specific EHEC strains with distinct serotypes, it is sufficient that a comparison is made between the obtained mass spectrometry data and reference data for the two specific EHEC strains with distinct serotypes. If the presence of an EHEC or identity of an EHEC is unknown, a comparison is advantageously made with reference data for a larger group of EHEC strains with distinct serotypes, e.g. 3, 4, 5, 6, 7, 8, 9 or 10 EHEC strains with distinct serotypes. The EHEC serotype is or the plurality of EHEC serotypes are preferably selected from the group consisting of *E*. *coli* O157H7, E. *coli* O26H11, *E. coli* O45H2, *E*. *coli* O103H2, *E*. *coli* O104H4, *E*. *coli* O111H8, *E*. *coli* O121H19, *E*. *coli* O145H28, and combinations thereof. In some preferred embodiments, the plurality of EHEC serotypes comprises *E*. *coli* O157H7, *E*. *coli* O26H11, *E. coli* O45H2, *E*. *coli* O103H2, *E*. *coli* O104H4, *E*. *coli* O111H8, *E*. *coli* O121H19 and *E*. *coli* O145H28.

The sequence of the obtained peptides, such as the obtained mass spectrometry data, may further be compared with non-EHEC bacteria, in particular non-EHEC *E*. *coli.* Hence, in some preferred embodiments, the reference data comprises information about the amino acid sequence of protein and/or peptides from non-EHEC strains. The non-EHEC strains can be any non-EHEC strain independent of its serotype. In preferred embodiments, the non-EHEC strain is a non-EHEC *E*. *coli* strain. The reference data for a non-EHEC strain can comprise reference data for one non-EHEC strains or for a combination of more than one non-EHEC strain. In preferred embodiments, the at least one non-EHEC is at least one Shiga toxin-producing *E*. *coli* (STEC). In some preferred embodiments, the reference data comprises information about the amino acid sequence of a plurality of peptides for a plurality, e.g. 2, 3, 4, 5, 6 or 7, non-EHEC strains, preferably a plurality, e.g. 2, 3, 4, 5, 6 or 7, of Shiga toxin-producing *E*. *coli* (STEC) strains.

The sequence of the obtained peptides, such as the obtained mass spectrometry data, and the reference data preferably provide the same type of information. In some preferred embodiments, the obtained mass spectrometry data comprise a mass spectrum or mass spectra. A mass spectrum is a histogram wherein intensity is plotted vs. mass-to-charge ratio (m/z). The x-axis of a mass spectrum typically represents the m/z ratio. The y-axis in of a mass spectrum typically represents the signal intensity of the ions. In preferred embodiments, the obtained mass spectrometry data comprise m/z values of peaks in the mass spectrum or mass spectra.

The mass spectrometry data obtained and used in the methods of the invention and the reference data preferably comprise proteomic profile data. The term "proteome" as used herein refers to the entire protein component that is or can be expressed by an organism, e.g. an EHEC or *E. coli* strain. The term "proteomic profile data" refers to information about the protein component of the organism, e.g. the EHEC or *E. coli* strain, characterised by the mass spectrum or mass spectra, in particular characterised by peaks in the mass spectrum or mass spectra, corresponding to the proteins making up the proteome of the organism or spectra, e.g. the EHEC or *E. coli* strain, present in the sample or the reference EHEC or *E. coli* data. The proteomic profile data comprise or are for instance m/z values of peaks in the mass spectrum or spectra. Hence, the reference data preferably comprises the amino acid sequences of protein and/or peptides of at least one EHEC strain with a specific serotype and optionally of one or more non-EHEC strains, preferably sequences of the proteome or part thereof.

In preferred embodiments the obtained mass spectrometry data, in particular proteomic profile data, comprise information about the amino acid sequence of a plurality of peptides in the sample. The proteomic profile data represent the entire proteome, in particular the enzymatically digested proteome, or a part thereof. That is, the mass spectrometry data, in particular proteomic profile data, may comprise information about part of amino acid sequences of peptides resulting from enzymatic digestion of bacterial protein, preferably of the proteome or part thereof. In particular the proteomic profile data allows to distinguish an EHEC strain with a specific serotype from another EHEC strains with a distinct serotype or an EHEC strain from non-EHEC strains. I.e., the proteomic profile data comprises information about the amino acid sequence of a sufficient number of peptides in order to distinguish an EHEC strain with a specific serotype from another EHEC strain, in particular with a specific, distinct serotype, or an EHEC strain from a non-EHEC strain.

Hence, the mass spectrometry data obtained and used in the methods of the invention, in particular proteomic profile data, preferably comprises information about the amino acid sequence of a plurality of peptides resulting from the enzymatic digestion of protein, preferably total protein, in the sample as described herein above.

Similarly, the reference data, in particular proteomic profile data, preferably comprises information about the amino acid sequence of protein and/or peptides for each EHEC strain, in particular with a specific serotype, and optionally non-EHEC strains, in particular of the proteome of each of such EHEC strains and optionally non-EHEC strains.

A "plurality of peptides" as used herein in the context of the cleavage step or the mass spectrometry data obtained in a method of the invention and the reference data preferably refers to at least 1000 peptides. There is no upper limit to the number of peptides. The upper limit is determined by the number of peptides resulting from the enzymatic digestion of the protein, preferably total protein, of the bacteria and the number of MS-spectra that can be interpreted in to a peptide sequence and may be up to e.g. 15000 peptides. A "plurality of peptides" may refer to at least 2000 peptides, 3000 peptides, 4000 peptides, 5000 peptides, 6000 peptides, 7000 peptides, 8000 peptides, 9000 peptides or 10000 peptides.

The reference data can be obtained or prepared by various methods that are part of the common general knowledge of a person skilled in the art. The references data can for instance comprise the sequences of proteins or the proteome of known bacterial strains. This is for instance described in Berendsen et al. 2017 and Berendsen et al. 2020, which use known proteomics data of different bacterial genus and species as reference data. The proteome of the EHEC strains described herein and of several non-EHEC strains has been determined and is available via the National Center for Biotechnology Information (NCBI) of the National Library of Medicine at the National Institutes of Health (NIH). The reference data for an EHEC strain, in particular of a specific serotype, preferably comprises the sequence of the proteome or part thereof of said EHEC strain. Similarly, the reference data of a non-EHEC strain or of non-EHEC strains preferably comprises the sequence of the proteome or part thereof of one or more non-EHEC strains.

Alternatively, a sample of peptides resulting from the enzymatic digestion, preferably by trypsin, of a culture of a known EHEC strain with a specific serotype or a non-EHEC strain can be obtained and such sample can be subjected to a cleavage step, and optionally mass spectrometry, preferably LC-MS/MS, as described herein. The obtained plurality of peptides or mass spectrum or mass spectra can be used as or to prepare reference data in accordance with the invention. As another alternative, an *in silico* enzymatic digestion can be carried out on the proteome of a known EHEC strain with a specific serotype or non-EHEC strain. In this case the peptides resulting from the *in silico* enzymatic digestion, in particular the sequences thereof, can be used as or to prepare reference data in accordance with the invention.

The obtained plurality of peptides, optionally mass spectrometry data, can be used to determine the sequence of the peptides in the sample, in particular of the peptides resulting from enzymatic digestion. Hence, in preferred embodiments, a method of the invention comprises determining the amino acid sequence of peptides resulting from enzymatic digestion of protein, preferably total protein, in the sample that is optionally subjected to mass spectrometry, preferably LC-MS/MS, in accordance with the invention based on the mass spectrometry data as defined herein. Using the m/z values of peaks in the mass spectrum or spectra, the amino acid sequences of the plurality of peptides can for instance be determined. Subsequently, the peptides can be compared with the reference data as defined herein. In preferred embodiments the mass spectrometry data obtained with a method of the invention comprises or is the amino acid sequences of a plurality of peptides as described herein above and the reference data comprise or are the amino acid sequence of protein of the at least one EHEC strain with a specific serotype, and optionally one or more non-EHEC strains. The comparison in this case comprises comparing the amino acid sequences of peptides in the sample with the amino acid sequences of protein in the reference data.

Software programs for protein identification or proteomics data analysis, using for instance mass spectrometry data, are available that can aid in comparing the sequences the obtained peptides with the sequences in the references data, such as PEAKS X (Bioinformatic Solutions Inc.) or MASCOT (Matrix Science).

Comparing is preferably made on the basis of the highest number of discriminatory peptides in the sample found in the reference data for an EHEC strain with specific serotype or optionally the non-EHEC strain(s). Preferably the comparison is made on the basis of the number of unique peptides in the sample that is identical to sequences in the reference data for an EHEC strain with a specific serotype or optionally the non-EHEC strain(s). As used herein "unique peptide" refers to a peptide that is only present in the reference data for a single EHEC or a non-EHEC and not in the reference data for another EHEC or a non-EHEC. Such unique peptides are therefore characteristic for a specific EHEC strain with a specific serotype or non-EHEC.

Hence, in some preferred embodiments, the reference data comprises information about the amino acid sequence of protein and/or peptides for each EHEC strain with a specific serotype and the comparing step comprises detecting the presence of absence of the obtained plurality of peptides with said amino acid sequence of a protein and/or peptides from each EHEC strain and optionally non-EHEC.

The presence or absence of an EHEC is preferably detected based on the number of unique peptides in the sample or the processed sample, such as peptides reflected by the obtained mass spectrometry data, that is identical to sequences of protein and/or peptides in reference data for an EHEC and/or non-EHEC. The higher the number of unique peptides in the sample that is identical sequences of protein and/or peptides in reference data for an EHEC strain with a specific serotype, the higher the chance that the relevant EHEC is present in the sample. I.e. if multiple unique peptides in the sample are identical to sequences of protein and/or peptides in reference data for an EHEC strain with a specific serotype, the presence of said EHEC in the sample is detected. If the number of peptides in the sample that is identical to sequences in protein and/or peptides in reference data for an EHEC strain with a specific serotype is low the absence of said EHEC in the sample is detected. Hence, in some preferred embodiments, the presence of an EHEC is detected and/or an EHEC strain with a specific serotype is identified if a plurality of unique peptides from an EHEC strain with a specific serotype in the reference data is detected in the sample.

In some preferred embodiments, a method of the invention comprises comparing the sequences of the obtained plurality of peptides, such as the obtained mass spectrometry data, with reference data for a plurality of EHEC strains with a specific serotypes, the reference data comprises information about the amino acid sequence of protein and/or peptides for each of the plurality of EHEC strains with a specific serotypes, and the presence of the EHEC is detected for which EHEC the highest number of peptides in the sample is identical to sequences of in protein and/or peptides in the reference data for that EHEC. Hence, in preferred embodiments, the presence of an EHEC is detected and/or an EHEC strain with a specific serotype is identified based on the highest number of unique peptides in the sample corresponding to sequences of protein and/or peptides of the reference data of an EHEC strain with a specific serotype.

In some preferred embodiments, the presence of an EHEC is detected if at least 5, preferably at least 10 peptides in the sample are identical to sequences of protein and/or peptides in the reference data for that EHEC. In this case, the number of peptides in the sample that are identical to sequences of protein and/or peptides in the reference data for other EHECs and non-EHEC *E*. *coli* is lower than 5, preferably 10. More preferably, the presence of an EHEC is detected if at least 15 peptides, such as at least 20, 30, 40, 50, 60, 70, 80, 90 or 100 peptides in the sample are identical to sequences of protein and/or peptidesin the reference data for that EHEC. In such case, the number of peptides in the sample that are identical to sequences of protein and/or peptides in the reference data for other EHEC and non-EHEC *E*. *coli* serotypes is lower than 15, 20, 30, 40, 50, 60, 70, 80, 90 or 100, respectively.

Identification of the EHEC can be performed in the same way as detection of the presence of an EHEC.

In particular, the EHEC is identified based on the number of unique peptides in the sample or processed sample, such as peptides reflected by the obtained mass spectrometry data, that is identical to sequences of protein and/or peptides, in reference data for an EHEC and/or non-EHEC. The higher the number of unique peptides in the sample, the higher the chance that the relevant EHEC is identified. I.e. if multiple unique peptides in the sample are identical to sequences of protein and/or peptides in reference data for an EHEC strain with a specific serotype, said EHEC is identified. If the number of unique peptides in the sample that is identical to sequences of protein and/or peptides in reference data for an EHEC strain with a specific serotype is low no EHEC is identified.

In some preferred embodiments, a method of the invention comprises comparing the sequences of the obtained plurality of peptides, such as the obtained mass spectrometry data, with reference data for a plurality of EHEC strains with a specific serotypes, the reference data comprises information about the amino acid sequence of protein and/or peptides for each of the plurality of EHEC strains with a specific serotypes, and the EHEC is identified for which the highest number of peptides in the sample is identical to sequences of in protein and/or peptides in the reference data for that EHEC. In such case, the plurality of EHECs is preferably at least 2, more preferably at least 3, more preferably at least 4, more preferably at least 5, more preferably at least 6, such as 7, 8 or 9. It is particularly preferred that the plurality of EHECs is preferably at least 7. In some preferred embodiments, the plurality of EHECs is 8. In some preferred embodiments, the plurality of EHECs is 9.

In some preferred embodiments, EHEC is identified if at least 5, preferably at least 10 peptides in the sample are identical to sequence of protein and/or peptides in the reference data for that EHEC. In this case, the number of peptides in the sample that are identical to sequence of protein and/or peptides for other EHECs and non-EHEC *E*. *coli* is lower than 5, preferably 10. More preferably, an EHEC is identified if at least 15 peptides, such as at least 20, 30, 40, 50, 60, 70, 80, 90 or 100 peptides in the sample are identical to sequence of protein and/or peptides in the reference data for that EHEC. In such case, the number of peptides in the sample that are identical to sequence of protein and/or peptides in the reference data for other EHEC and non-EHEC *E*. *coli* strains is lower than 15, 20, 30, 40, 50, 60, 70, 80, 90 or 100, respectively.

A method of the invention can be performed without the need to first identify the bacterial genus or species present in the sample.

However, it is also possible that a method of the invention is preceded by a method for identifying a bacterial genus and/or species in the sample, and a method of the invention is performed if an *Escherichia* species, in particular *E*. *coli,* is identified in the sample. Hence in some embodiments, a method of the invention comprises a step of identifying a bacterial genus and/or species in the sample. Identification of a bacterial genus and/or species is for instance performed with mass spectrometry, in particular LC-MS/MS. Such methods for identification of a bacterial genus and species in a sample are for instance described in Berendsen et al. 2017 and Berendsen et al. 2020.

Figure 2 shows a schematic presentation of data analysis in a method for identifying EHEC in accordance with the present invention, preceded by genus and species identification step. A file with peptides is generated with PEAKS X or another program. The peptides can be preprocessed e.g. by replacing Isoleucines to Leucines (to avoid misinterpretation because these residues have the same mass) and/or removing the duplicate peptides. After this, a genus search is performed. In this search, input peptides from the sample are searched for in proteomes of all genera in the reference database. If a match is identified for a genus, the match is determined to be discriminative (only present in this genus) or not discriminative for this genus. After the genus search, all genera are selected with at least 3 genus discriminative peptides. In each genus separate, a species search is then performed. In this species search, all identified peptides for this genus present in the sample are searched for in proteomes of the species present in the reference database that belongs to this genus. If a match is identified for a species, it is determined to be discriminative (only present in this species) or not discriminative for this species within the genus that is selected. A final species is then selected based on the number of species discriminative peptides (and if necessary also by the number of total identified peptides for this species).

An output table can be generated, which contains the number of species discriminative (unique) peptides and the total number of species identified peptides for each selected genus. If the species is identified as a *E. coli* the EHEC detection is executed. In this example search all input peptides are searched for in proteomes of all 10 entries (9 entries with proteomes of a EHEC strain (O157, O26, O45v1, O45v2, 0103, 0104, O111, 0121, 0145) and one entry with representative proteomes of other *E*. *coli* isolates (*E*. *coli* [non-EHEC]). If a match is identified for an entry, it is determined to be discriminative (only present in this entry) or not discriminative for this entry within the P4ED reference database. A final entry is then selected based on the number of entry discriminative peptides (and if necessary also by the number of total identified peptides for this entry). An output table can be generated, which contains the number of entry discriminative (unique) peptides and the total number of entry identified peptides for the identified entry.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

Figure 1: Schematic presentation of the workflow of a Mass Spectrometry based analysis. Using an universal sample preparation method for shotgun proteomics SP3, (Single-Pot Solid-Phase-enhanced Sample Preparation' sample preparation SP3) is used to prepare a trypsin digest for mass spectrometry measurement (Hughes et al. 2014). Next, peptide mix is offered to the LC-MS/MS where the "data acquisition" takes place. MS-spectra are generated from the peptides and peptides can be identified using a software program and a database that contains the proteins of in the analysed sample.
Figure 2: Schematic presentation of data analysis in a method for identifying EHEC, preceded by genus identification step.

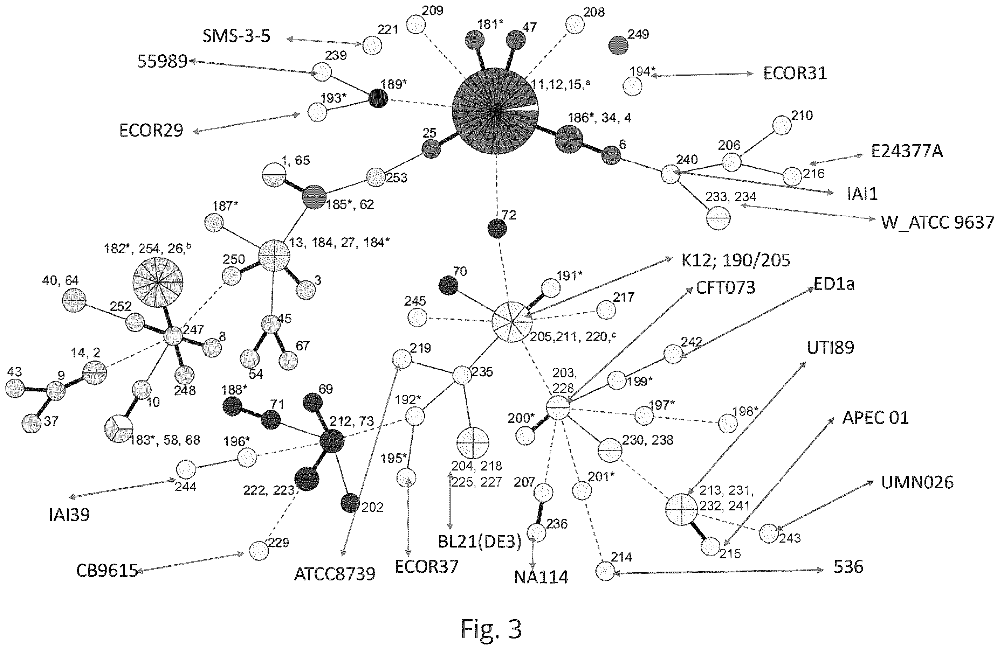

Figure 3: Is based on Figure 1 from the article "Rapid and reliable discrimination between *Shigella* species and *Escherichia coli using* MALDI-TOF mass spectrometry" by Paauw et al. 2015. Minimum spanning tree created from 146 isolates from the *E.coli*-Shigella species population based on MLVA data. Single locus variants are connected by a thick line, double locus variants are connected by a thin line, and triple locus variants are depicted by a dashed line. Isolates identified as *S. sonnei* (red), *S. flexneri* (green), *S. boydii* (turquois) *S. dysenteriae* (pink), *E*. *coli* (yellow), *E*. *coli* O104H4 (dark blue) and *E*. *coli* O157:H7 (purple) are indicated. Numbers in the figure correspond to the strain numbers in Table S1 in the article; ^{a} indicates strain numbers 5, 11, 12, 15, 16, 17, 18, 19, 20, 21, 22, 23, 24, 28, 29, 30, 31, 32, 33, 35, 36, 38, 42, 48, 49, 52, 55, 56, 59, 60, 61, 63, 66, and 251; ^{b} indicates strain numbers 26, 39, 41, 44, 46, 50, 51, 53, 57, 182, and 254; ^{c} indicates strain numbers 205, 211, 220, 224, 226, 237, and 246; isolates marked with an asterisk are the isolates selected for construction of the Shig/Ecoli MALDI-TOF MS library.

Added to the picture are isolates number of the *E*. *coli* of which the proteome is included in the non-EHEC entry in the database; EHEC_db27.

### Examples

### Materials and methods

### Bacterial strains

A total of 59 *E. coli* strains were included in the study. Twenty-seven *E. coli* strains originated from the ECOR collection, which are all not EHEC strains (Ochman et al. 1984). Next, 32 EHECs (12 *E. coli* O157:H7, 2 *E. coli* O104:H4, 3 *E. coli* O103:H2, 3 *E. coli* O111:H8, 3 *E. coli* O121:H19, 3 *E. coli* O145:H28, 3 *E. coli* O26:H11, 3 *E. coli* O45:H2) were obtained from the Dutch Centre for Infectious Disease Control, National Institute for Public Health and the Environment (RIVM). Identification of EHEC and non-EHEC *E. coli* isolates was based biochemical and whole genome characterization.

### Culture conditions

From the -80°C stock, the *E. coli* isolates were recovered on blood agar plates and cultured 18- 24 h at 35°C. A colony was picked and streaked on a new blood agar plate. The sample preparation was executed direct from colonies isolated from the second blood agar plate that was incubated for maximal 24 h at 35°C.

### Sample preparation for LC-MS/MS analysis

Sample preparation was executed with a modified SP3 protocol (Hayoun et al. 2019). From the blood agar culture plate, a small sample of the culture material was resuspended in a tube with 500 µL lysis buffer (4% SDS, 100 mM DTT, 100 mM Tris-HCl, pH 8). Incubate 1 h at 95°C. Subsequently, the sample was sonicated in a ultrasonic bath (Crest Ultrasonics) for 5 min, followed by adding 40 µl solution of Sera-Magnetic beads (25 µg/µl of hydrophilic beads and 25 µg/µl of hydrophobic beads). After gently mixing, 500 µl 100% ethanol was added and thereafter incubated for 5 min at 900 rpm on a thermomixer (Thermomixer, Eppendorf). Sera-Magnetic beads were retained in solution by a neodymium magnet (MagRack 6 Cytiva product no. 28-9489-64), while the liquid was removed by pipetting. Next, Sera-Magnetic beads were washed 3 times with 180 µl 80% ethanol whereby the beads were kept in the reaction tube using the neodymium magnet. After removal of the 80% ethanol after the third wash 100 µL digestion buffer (1 µg/µl Trypsin Gold (Promega) in 50 mM ABC-buffer) was added, sonicated 30 s and mixed gently to disperse the beads homogenous in the solution and incubated for 15 min at 50 °C. Digestion reaction was stopped by adding 5 µl 10% TFA. Samples where filtered with AeroPrep^{™} Advance (PN 518-0022) according manufacturer guidelines. From each sample the protein concentration was measured using NanoDrop Protein A280 measurement application. Sample was diluted to a final concentration of 0.2 mg/ml.

### LC-MS/MS analysis

Five µl of 0.2 mg/ml sample was injected in to a Ultra-High-Performance Liquid Chromatography (UHPLC; UltiMate 3000 Rapid Separation LCnano System, Thermo Fisher). The analysis of the protein digests was performed using a C18 column (PepMap 100 C18 5µm 0.3x5 mm, Thermo Scientific). The samples were washed for 8 minutes with 0.1% TFA before switching the trap column online with the analytical column. Peptides were separated using the C18 column (C18 PepMap 75µm IDx250mm) with a sustained flow of 0.3 µl/min. The consistency of the mobile phases was 0.1% FA in water (eluent A) and 0.1% FA in acetonitrile (ACN) (eluent B) with the following LC gradient for the digested samples (120 min. run): the gradient started at 96% eluent A and went to 38% eluent B in 90 minutes, followed by a step from 38-80% B in 2 minutes, followed by a 10 minute hold. Following that, eluent B was ramped down to 4% in 1 minute and held at 4% for 19 minutes. The UHPLC was connected to the Q Exactive Plus (Q Exactive Plus Orbitrap^{™} Mass Spectrometer, Thermo Scientific) using the EASY-Spray Ion Source (Thermo Fisher). The EASY-Spray column (Acclaim PepMap RSLC C18 2µm, 100Å, 75 µm × 25 mm) was used with a flow rate of 30 nL/min and a column temperature of 37°C.

### MS/MS data analysis

The recorded MS-spectra were analyzed and compared to the EHEC_db27 database. Briefly, this database contains annotated protein sequences in FASTA format that were derived from sequenced genomes on the NCBI FTP server. The database contains 10 entries, representing non-EHEC and 8 EHEC strains (*E.coli* O157:H7, *E.coli* O26:H11, *E.coli* O45:H2st20, O45:H2st165, *E.coli* O103:H2, *E. coli* O104:H4, *E.coli* O111:H8, *E.coli* O121:H19 and *E.colε* O145:H28. The MS spectra obtained from each sample were assigned to peptides using PEAKS X (Bioinformatics Solutions Inc., Waterloo, Canada) and the custom database (EHEC_db27) (Zhang et al. 2012). Furthermore, filters were used to exclude duplicate peptides and advanced missed cleavage handling (Berendsen et al. 2020; Balvers et al. 2023; Paauw et al. 2023).Only peptides with a false discovery rate (FDR) ≤ 0.1 % were used for further analysis.

### EHEC Protein database development

The EHEC_db27 database contains the annotated protein sequences of the different isolates in the database. For each EHEC entry the unique proteins of two isolates of a EHEC strain are included, except for EHEC O45H2st20 and O45H2st165 these contained the unique proteins of 8 and 3 isolates, respectively. To have a representative entry for the *E. coli* that are not EHEC isolates (non-EHEC) the unique proteins of 21 *E. coli* isolates are included (see figure 3). These isolates were selected as a representative set for the proteomics diversity in non-EHECs based on MLVA results (Paauw et al. 2015).

### Peptide-based EHEC analysis

The developed application proteome2EHEC processes a list of peptides (which can be in a .csv or .txt format and independent of the used software application for peptide assignment). The peptide lists are "cleaned" (All isoleucine (I) amino acid residues in the input peptides with leucine (L) before analysis and duplicate input peptides, including duplicates caused by the "I-to-L" replacement, are removed). Subsequently, the analysis searches for matches between all input peptides and all entries in the EHEC_db27 database. For each peptide with a match, it determines for which entry the peptide has a "hit" (i.e. a perfect match for a reference sequence of an entry in EHEC_db27) and also whether the peptide is a "entry discriminative hit" (i.e. whether the peptide matches one of the 10 entries in the EHEC_db27 database and for that reason is discriminative). The tested colony is identified as one of the 9 EHEC entries or as a *E. coli* (non-EHEC) based on the entry with the highest number of discriminative peptides (unique peptides).

### Results

To validate the developed method to detect and characterize if an EHEC (E. *coli* O157:H7, *E. coli* O104:H4, *E. coli* O103:H2, *E. coli* O111:H8, *E. coli* O121:H19, *E. coli* O145:H28, *E. coli* O26:H11, *E. coli* O45:H2) is in the measured sample or not, 59 *E. coli* well characterized isolates were tested. All 27 *E. coli* isolates which weren't an EHEC were identified as a *"E. coli* non-EHEC" and all 32 EHEC were identified as a EHEC with the correct serotype based on the highest number of unique peptides identified in each entry of the database (Table 1).

### References

Balvers M, Gordijn IF, Voskamp-Visser I, Schelling MFA, Schuurman R, Heikens E, et al. Proteome2virus: Shotgun mass spectrometry data analysis pipeline for virus identification. Journal of Clinical Virology Plus. 2023;3(2).
Berendsen EM, Levin E, Braakman R, der Riet-van Oeveren DV, Sedee NJ, Paauw A. Identification of microorganisms grown in blood culture flasks using liquid chromatography-tandem mass spectrometry. ture Microbiol. 2017 Oct;12:1135-1145. doi: 10.2217/fmb-2017-0050.
Berendsen EM, Levin E, Braakman R, Prodan A, van Leeuwen HC, Paauw A. Untargeted accurate identification of highly pathogenic bacteria directly from blood culture flasks. Int J Med Microbiol. 2020 Jan;310(1):151376. doi: 10.1016/j.ijmm.2019.151376.
Hayoun K, Gouveia D, Grenga L, Pible O, Armengaud J, Alpha-Bazin B. Evaluation of Sample Preparation Methods for Fast Proteotyping of Microorganisms by Tandem Mass Spectrometry. Front Microbiol. 2019 Sep 6; 10: 1985.
Hughes CS, Foehr S, Garfield DA, Furlong EE, Steinmetz LM, Krijgsveld J, Ultrasensitive proteome analysis using paramagnetic bead technology. Mol Syst Biol. 2014 Oct 30;10(10):757. doi: 10.15252/msb.20145625.
Ochman H, Selander RK. Standard reference strains of Escherichia coli from natural populations. J Bacteriol. 1984 Feb;157(2):690-3.
Paauw A, Jonker D, Roeselers G, Heng JM, Mars-Groenendijk RH, Trip H, Molhoek EM, Jansen HJ, van der Plas J, de Jong AL, Majchrzykiewicz-Koehorst JA, Speksnijder AG. Rapid and reliable discrimination between Shigella species and Escherichia coli using MALDI-TOF mass spectrometry. Int J Med Microbiol. 2015 Jun-Aug;305(4-5):446-52. doi: 10.1016/j.ijmm.2015.04.001.
Paauw A, Scholz HC, Mars-Groenendijk RH, Dekker LJM, Luider TM, van Leeuwen HC. Expression of virulence and antimicrobial related proteins in Burkholderia mallei and Burkholderia pseudomallei. PLoS Negl Trop Dis. 2023 Jan 6;17(1):e0011006.
Zhang J, Xin L, Shan B, Chen W, Xie M, Yuen D, et al. PEAKS DB: de novo sequencing assisted database search for sensitive and accurate peptide identification. Mol Cell Proteomics. 2012 Apr;11(4):M111.010587.

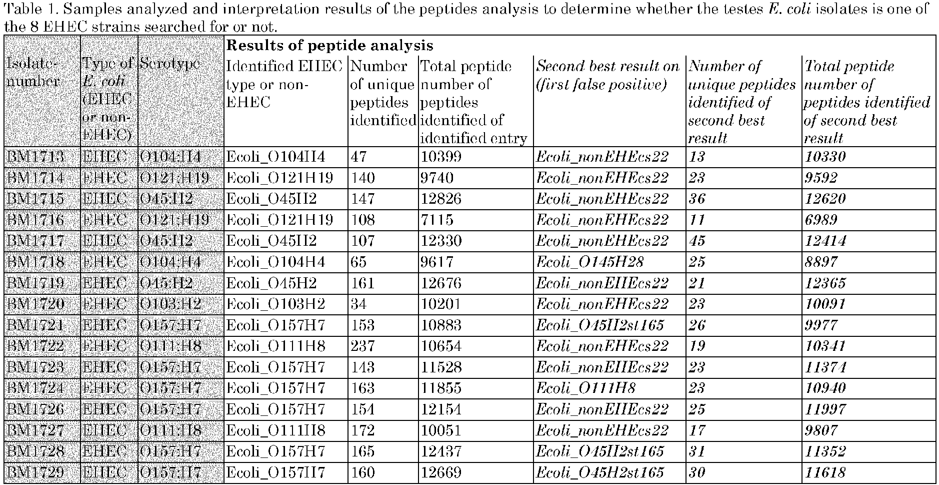

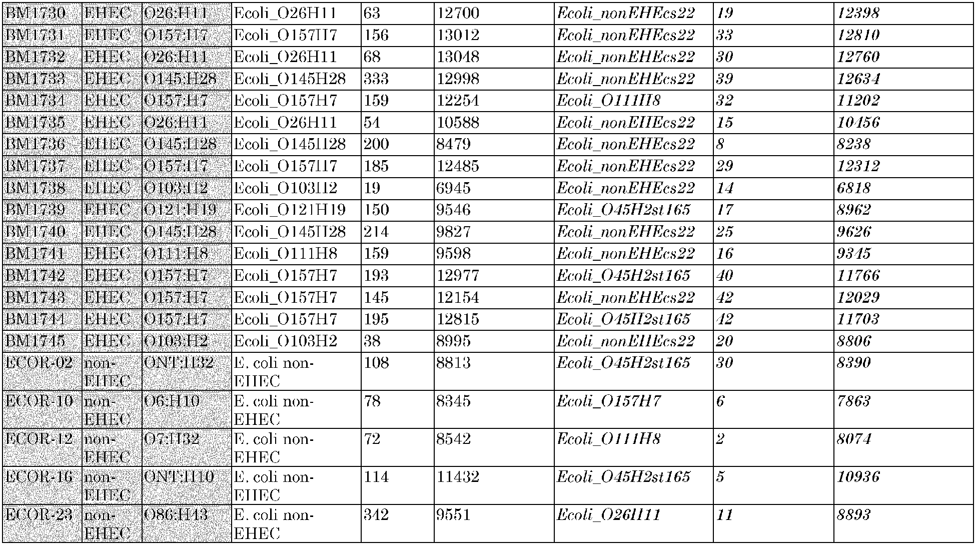

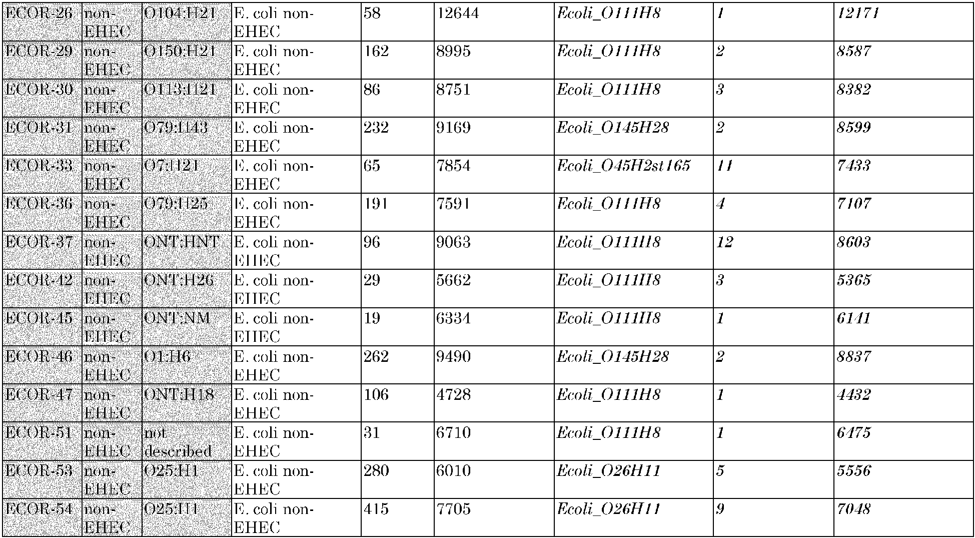

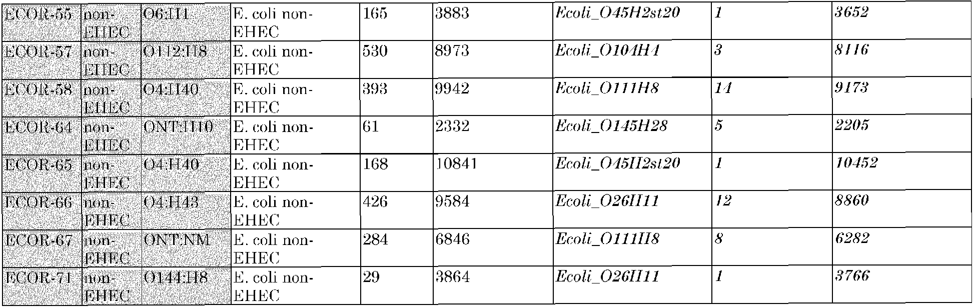

## Claims

1. A method for detecting the presence or absence of an enterohemorrhagic *Escherichia coli* (EHEC) in a sample, comprising:
- subjecting the sample or protein present therein to a cleavage step to obtain a plurality of peptides,
- comparing the sequences of the obtained peptides with reference data for at least one EHEC strain with a specific serotype, and optionally one or more non-EHEC strains, and
- detecting the presence or absence of an EHEC based on the comparison.

2. The method according to claim 1 wherein the sample or protein therein or the plurality of peptides is subjected to mass spectrometry to obtain mass spectrometry data and the obtained mass spectrometry is compared with the reference data.

3. The method according to claim 2, wherein the mass spectrometry data comprise information about the amino acid sequence of the plurality of peptides and the reference data comprises information about the amino acid sequence of protein and/or peptides for each of the at least one EHEC strain with specific serotype and optionally the one or more non-EHEC strains.

4. The method according to any one of the preceding claims, wherein said comparing comprises comparing the sequences of the obtained peptides and/or the obtained mass spectrometry data with reference data for a plurality of EHEC strains with a specific serotype and the reference data comprises information about the amino acid sequence of protein and/or peptides for each of the plurality of EHEC serotypes and optionally the one or more non-EHEC strains.

5. The method according to any one of the preceding claims, wherein the plurality of peptides for each of the EHEC strains with specific serotypes represents the proteome of the EHEC strain with specific serotype.

6. The method according to any one of the preceding claims, wherein
the reference data comprises information about the amino acid sequence of protein and/or peptides for at least one non-EHEC *E. coli* strain, preferably at least one Shiga toxin-producing *E. coli* (STEC) strain.

7. The method according to any one of claims 2-5 wherein said plurality of peptides comprises at least 500, preferably at least 1000, more preferably at least 2000, peptides for each EHEC strain with a specific serotype and each non-EHEC strain.

8. The method according to any one of the preceding claims, wherein the method comprises identifying the EHEC present in the sample.

9. The method according to any one of the preceding claims, wherein the reference data comprises information about the amino acid sequence of protein and/or peptides for each EHEC strain with a specific serotype and optionally non-EHEC strain, and the comparing step comprises detecting the presence of absence of the sequences of the obtained plurality of peptides with said amino acid sequence of protein and/or peptides for each EHEC strain and optionally non-EHEC strain.

10. The method according to any one of the preceding claims, wherein the presence of an EHEC is detected and/or an EHEC strain with a serotype is identified if a plurality of peptides from an EHEC strain with a specific serotype in the reference data is detected in the sample.

11. The method according to any one of the preceding claims, wherein the presence of an EHEC is detected and/or an EHEC strain with a specific serotype is identified based on the highest number of unique peptides in the sample corresponding to sequences of protein and/or peptides of the reference data of an EHEC strain with a specific serotype.

12. The method according to any one of the preceding claims, wherein the EHEC strains with a specific serotype or serotypes is/are selected from the group consisting of *E*. *coli* O157H7, *E. coli* O26H11, *E. coli* O45H2, *E. coli* O103H2, *E. coli* O104H4, *E. coli* O111H8, *E. coli* O121H19 and *E. coli* O145H28, and combinations thereof.

13. The method according to any one of the preceding claims, wherein the mass spectrometry is liquid chromatography tandem mass spectrometry (LC-MS/MS).

14. Use of mass spectrometry, preferably liquid chromatography tandem mass spectrometry (LC-MS/MS), to detect the presence or absence of and/or to identity an enterohemorrhagic *Escherichia coli* (EHEC) in a sample, preferably wherein the sample is subjected to mass spectrometry to obtain mass spectrometry data and the obtained mass spectrometry data is compared with reference data for at least one EHEC strain with a specific serotype and optionally one or more non-EHEC strains.
